# EUROPÄISCHE PATENTSCHRIFT

(11) **EP 1 734 883 B1**
(45) Veröffentlichungstag und Bekanntmachung des Hinweises auf die Patenterteilung: **06.09.2017**
(21) Anmeldenummer: 05735337.7
(22) Anmeldetag: 14.04.2005
(51) Int. Cl.: A61B 50/30, A61L 2/26, A61L 2/02, A61B 50/31

(54) **STERILBEHÄLTER**
STERILE CONTAINER COMPRISING A STERILE FILTER
CONTENANT DE STÉRILISATION

(30) Priorität: 16.04.2004 DE 102004020803
(43) Veröffentlichungstag der Anmeldung: 27.12.2006
(73) Patentinhaber: Aesculap AG, 78532 Tuttlingen (DE)
(72) Erfinder: JAKAB, Mariana, 78532 Tuttlingen (DE); OERTMANN, Friedrich-Wilhelm, 78532 Tuttlingen (DE); SCHUSTER, Stefan, 78048 Villingen-Schwenningen (DE); RENNER, Torsten, 07607 Eisenberg (DE)
(74) Vertreter: Hoeger, Stellrecht & Partner Patentanwälte mbB
(86) Internationale Anmeldenummer: PCT/EP2005/003939
(87) Internationale Veröffentlichungsnummer: WO 2005/099610

(56) Entgegenhaltungen:
- EP-B- 1 098 669
- WO-A-93/20853
- WO-A-99/27969
- DE-A1- 10 210 905
- DE-U1- 20 118 911

## Beschreibung

Die Erfindung betrifft einen Sterilbehälter, insbesondere zur Aufnahme und sterilen Aufbewahrung von chirurgischem Besteck oder chirurgischem Material, mit einem durch einen Behälterboden und Behälterwände gebildeten Aufnahmeraum, mit einem Deckel zum Verschließen des Aufnahmeraums, mit einer Gasaustauschöffnung zum Bereitstellen einer Fluidverbindung zwischen dem Aufnahmeraum und einer Umgebung des Sterilbehälters, mit einer ein erstes und ein zweites Halteteil umfassenden Filterhalterung und mit einem Sterilfilter, wobei das Sterilfilter in einer Sterilstellung, in welcher das Sterilfilter die Gasaustauschöffnung verschließt, zwischen dem ersten und dem zweiten Halteteil gehalten ist, wobei das Sterilfilter durchbrechungsfrei ausgebildet ist, wobei das erste Halteteil mindestens eine erste Haltefläche aufweist, wobei das zweite Halteteil mindestens eine zweite Haltefläche aufweist und wobei das Sterilfilter zwischen der ersten und der zweiten Haltefläche in der Sterilstellung klemmend gehalten ist, wobei mindestens ein Überdruckventil zum Bereitstellen einer Fluidverbindung zwischen einer Umgebung und dem Aufnahmeraum des Sterilbehälters vorgesehen ist und daß das Überdruckventil eine Ventilöffnung und eine Ventilklappe zum Öffnen und Schließen der Ventilöffnung umfaßt, wobei das Überdruckventil derart ausgebildet ist, daß in einer Grundstellung die Ventilöffnung geschlossen ist und daß in einer Durchlaßstellung bei Überschreiten einer Mindestdruckdifferenz zwischen im Aufnahmeraum und in der Umgebung des Sterilbehälters herrschenden Drücken die Ventilöffnung mindestens teilweise geöffnet ist.

Sterilfilter für Sterilbehälter der eingangs beschriebenen Art sind üblicherweise in Form einer flachen kreisförmigen Filterscheibe ausgebildet, welche eine konzentrische Bohrung aufweist und die Gasaustauschöffnung verschließt. Die Gasaustauschöffnung ermöglicht den Austausch von Fluiden, also insbesondere Gasen, Flüssigkeiten oder Gas-Flüssigkeits-Gemischen, zwischen der Umgebung und dem Aufnahmeraum des Sterilbehälters. Zum Befestigen des Filters am Sterilbehälter ist es bekannt, am Deckel einen Befestigungsstift vorzusehen, welcher die Bohrung der Filterscheibe durchsetzt und so das Filter gegen eine seitliche Bewegung sichert. Zusätzlich greift an dem Befestigungsstift üblicherweise ein federnder Mechanismus an, der das Filter gegen eine Wand des Containers drückt, beispielsweise eine Wand des Deckels. Nachteilig bei dieser Art der Filterhalterung ist, daß zwei konzentrische Dichtflächen bereitgestellt werden müssen, und zwar eine direkt um die konzentrische Bohrung des Sterilfilters, die zweite am äußeren Rand des Filters. Zudem kann es bei wiederverwendbaren Filtern vorkommen, daß beim Entnehmen des Filters zu Reinigungszwecken das Filter an der Bohrung einreißt. Dadurch wird das Filter völlig unbrauchbar und muß ersetzt werden.

Aus der DE 20118911 U1 ist ein Sterilbehälter bekannt, welcher eine vom Behälter lösbare Filterkassette mit einem Träger, einem Halteteil und einem zwischen dem Träger und dem Halteteil gehaltenen Sterilfilter umfasst. In der WO 93/20853 A1 ist eine Vorrichtung zum Halten eines medizinischen Instruments offenbart. Ferner ist in der WO 99/27969 A2 ein Sterilisierbehälter beschrieben. Ein weiterer Sterilbehälter ist aus der DE 102 10 905 A1 bekannt. Und schließlich ist in der EP 1 098 669 B1 eine tellerförmige Filterhalterung für einen Sterilisierbehälter offenbart.

Es ist daher Aufgabe der vorliegenden Erfindung, einen Sterilbehälter der eingangs beschriebenen Art so zu verbessern, daß der Aufbau desselben vereinfacht wird.

Diese Aufgabe wird bei einem Sterilbehälter der eingangs beschriebenen Art erfindungsgemäß dadurch gelöst, daß das erste Halteteil, das zweite Halteteil und das Sterilfilter miteinander lösbar verbindbar sind, daß eine Wandfläche des Sterilbehälters einstückig mit der mindestens einen ersten Haltefläche ausgebildet ist, daß das zweite Halteteil relativ zum ersten Halteteil beweglich gelagert ist, daß Lagerungselemente am zweiten Halteteil und am Sterilbehälter benachbart der Gasaustauschöffnung zur beweglichen Lagerung des zweiten Halteteils am Sterilbehälter vorgesehen sind und daß das Sterilfilter in der Schließstellung zwischen der mindestens einen ersten und der mindestens einen zweiten Haltefläche unter Vorspannung gehalten ist.

Dadurch, daß das Sterilfilter durchbrechungsfrei ausgebildet ist, wird kein Befestigungsstift mehr benötigt und zudem kann auf eine zweite Abdichtung des Sterilfilters gegenüber einer Wand des Sterilbehälters verzichtet werden. Zur Befestigung des Sterilfilters reicht es daher beispielsweise aus, das Sterilfilter zwischen zwei die Gasaustauschöffnung umgebenden Halteflächen, beispielsweise zwei Ringflächen, klemmend zu halten. Die Filterhaltung wird in ihrem Aufbau noch weiter vereinfacht, daß eine Wandfläche des Sterilbehälters einstückig mit der mindestens einen ersten Haltefläche ausgebildet ist. Beispielsweise läßt sich so die mindestens eine erste Haltefläche direkt in die Wandfläche des Sterilbehälters integrieren, beispielsweise in eine Deckelfläche. Gemäß der Erfindung ist vorgesehen, daß mindestens ein Überdruckventil zum Bereitstellen einer Fluidverbindung zwischen einer Umgebung und dem Aufnahmeraum des Sterilbehälters vorgesehen ist, daß das Überdruckventil eine Ventilöffnung und eine Ventilklappe zum Öffnen und Schließen der Ventilöffnung umfaßt, wobei das Überdruckventil derart ausgebildet ist, daß in einer Grundstellung die Ventilöffnung geschlossen ist und daß in einer Durchlaßstellung bei Überschreiten einer Mindestdruckdifferenz zwischen im Aufnahmeraum und in der Umgebung des Sterilbehälters herrschenden Drücken die Ventilöffnung mindestens teilweise geöffnet ist. Durch das Überdruckventil läßt sich eine Beschädigung des Sterilbehälters vermeiden, da bei Druckdifferenzen, die größer sind als die Mindestdruckdifferenz, ein Gasaustausch durch das Sterilfilter hindurch nicht ausreicht, um diese Druckdifferenz abzubauen und der Sterilbehälter insgesamt so zusammengedrückt oder ausgewölbt werden kann. Vorteilhafterweise ist das zweite Halteteil relativ zum ersten Halteteil beweglich gelagert. Beispielsweise kann dann eines der beiden Halteteile das Sterilfilter stützen und/oder zusammen mit diesem bewegt werden, wenn dieses als Ventilklappe dient. Auf besonders einfache Weise läßt sich eine bewegliche Lagerung zwischen dem ersten und dem zweiten Halteteil dadurch realisieren, daß Lagerungselemente am zweiten Halteteil und am Sterilbehälter benachbart der Gasaustauschöffnung zur beweglichen Lagerung des zweiten Halteteils am Sterilbehälter vorgesehen sind. Vorteilhafterweise ist das Sterilfilter in der Grundstellung zwischen der mindestens eine ersten und mindestens einen zweiten Haltefläche unter Vorspannung gehalten. Dadurch wird sichergestellt, daß ein Gasaustausch in der Schließstellung nur durch das Sterilfilter hindurch zwischen dem Aufnahmeraum und der Umgebung des Sterilfilters gestattet wird.

Vorteilhaft ist es, wenn die mindestens eine erste und die mindestens eine zweite Haltefläche einander gegenüberliegend angeordnet sind. Dies bedeutet, daß die zwei Halteflächen direkt aneinander anliegen würden, wenn nicht das Sterilfilter zwischen ihnen klemmend gehalten wäre. Dadurch können die beiden Halteflächen sehr klein ausgebildet werden, so daß eine möglichst große Filterfläche frei bleibt. Trotzdem wird das Sterilfilter sicher gehalten.

Besonders einfach wird der Aufbau des Sterilbehälters, wenn das Sterilfilter und/oder die Gasaustauschöffnung kreisförmig oder im wesentlichen kreisförmig ausgebildet sind.

Vorteilhaft ist es, wenn die Gasaustauschöffnung am Deckel vorgesehen ist und wenn die Wandfläche einen Teil des Deckels des Sterilbehälters bildet. Dies erleichtert das Austauschen des Sterilfilters, da auch bei einem gefüllten Aufnahmeraum das Sterilfilter nach Entfernen des Deckels auf einfache Weise ausgewechselt werden kann.

Günstigerweise umfaßt das erste Halteteil eine Sterilfilteraufnahme, welche eine Querschnittsfläche aufweist, die im wesentlichen zu einer Filterfläche des Sterilfilters korrespondiert. Das Sterilfilter kann in die Sterilfilteraufnahme eingelegt werden und wird dadurch in dieser beispielsweise auch seitlich geführt.

Besonders einfach wird der Aufbau des Sterilbehälters, wenn die Sterilfilteraufnahme topfartig ausgebildet ist. Das Sterilfilter wird in der topfartigen Sterilfilteraufnahme seitlich geführt. Insbesondere dann, wenn die Gasaustauschöffnung in einem Boden der topfartigen Sterilfilteraufnahme vorgesehen ist, kann eine verbleibende Bodenfläche der Filteraufnahme die mindestens eine erste Haltefläche bilden.

Günstig ist es, wenn in einer Seitenwand der Sterilfilteraufnahme Durchbrechungen vorgesehen sind. Durch die Durchbrechungen kann ein Gasaustausch stattfinden, wenn beispielsweise das Sterilfilter parallel zu einer Bodenfläche der Filteraufnahme von dieser weg bewegt wird und so die Filteraufnahme auch nach einem Abheben von der Bodenfläche verschließen würde. Insbesondere können die Durchbrechungen über einen Umfang einer Seitenwand einer topfartigen Sterilfilteraufnahme gleichmäßig verteilt sein, beispielsweise in Form von parallel zur Bodenfläche verlaufenden Schlitzen.

Vorteilhafterweise ist mindestens ein Stützelement vorgesehen, welches die mindestens eine erste Haltefläche oder die mindestens eine zweite Haltefläche umfaßt und welches die Gasaustauschöffnung mindestes teilweise überdeckende Auflageflächen zum einseitigen Stützen des Sterilfilters umfaßt. Das mindestens eine Stützelement stellt sicher, daß bei großen Druckdifferenzen zwischen dem Aufnahmeraum und einer Umgebung des Sterilbehälters ein Durchwölben des Sterilfilters eingeschränkt und minimiert wird, wodurch sich Beschädigungen des Sterilfilters vermeiden lassen.

Auf besonders vorteilhafte Weise läßt sich das Sterilfilter abstützen, wenn das mindestens eine Stützelement ein von einem Zentrum der Gasaustauschöffnung ausgehender radialer Stützsteg ist. Mit diesem läßt sich die Gasaustauschöffnung auf einfache Weise überspannen und beispielsweise in unterschiedliche Segmente aufteilen, so daß einzelne kleine Sterilfilterflächenbereiche verbleiben, die auch größeren Druckdifferenzen zwischen dem Aufnahmeraum und der Umgebung des Sterilbehälters standhalten können.

Vorteilhaft kann es auch sein, wenn das mindestens eine erste Stützelement ein zur Gasaustauschöffnung konzentrischer Stützring ist. Dadurch läßt sich die freie Sterilfilterfläche optimieren, da ein konzentrischer Stützring beispielsweise die Gasaustauschöffnung umgebend vorgesehen sein kann.

Um das die Gasaustauschöffnung überdeckende Sterilfilter sicher zu halten, ist es günstig, wenn zwei oder drei konzentrische Stützringe vorgesehen sind.

Damit ein Eindringen beispielsweise von Luft in den Aufnahmeraum des Sterilbehälters im Bereich der am Sterilfilter anliegenden Halteflächen vermieden werden kann, ist es vorteilhaft, wenn das mindestens eine erste und/oder das mindestens eine zweite Halteteil eine Dichtung tragen, an welcher das Sterilfilter in der Sterilstellung anliegt. Ein Gasaustausch zwischen dem Aufnahmeraum und einer Umgebung des Sterilbehälters kann dann nur noch durch das Sterilfilter hindurch erfolgen.

Damit die Dichtung nicht in unbeabsichtigter Weise verrutschen kann, ist es günstig, wenn das mindestens eine erste und/oder das mindestens eine zweite Halteteil eine Dichtungsaufnahme aufweisen, in welche die Dichtung mindestens teilweise eintaucht.

Ein besonders einfacher Aufbau des Sterilbehälters ergibt sich, wenn die Dichtung in Form eines Dichtringes ausgebildet ist.

Um ein Auswechseln des Sterilfilters zu erleichtern, ist es günstig, wenn das erste Halteteil und das zweite Halteteil miteinander lösbar verbindbar sind.

Gemäß einer bevorzugten Ausführungsform der Erfindung kann ferner vorgesehen sein, daß das Sterilfilter und die Filterhalterung lösbar verbindbar sind, daß das Sterilfilter in einer Entnahmestellung von der Filterhalterung lösbar und in einer Verbindungsstellung an der Filterhalterung gehalten ist. Auf diese Weise läßt sich das Sterilfilter, beispielsweise zu Reinigungszwecken oder zur Entsorgung, aus der Filterhalterung lösen.

Eine besonders sichere Verbindung zwischen den beiden Halteteilen kann hergestellt werden, wenn eine Bajonettverbindung zum Verbinden des ersten Halteteils mit dem zweiten Halteteil und zum Überführen der Filterhalterung von der Entnahmestellung in die Verbindungsstellung vorgesehen ist. Beispielsweise lassen sich die beiden Halteteile relativ zueinander in einer Kupplungsrichtung in Eingriff bringen und durch Verdrehen in einer Ebene quer zur Kupplungsrichtung relativ zueinander in die Verbindungsstellung überführen.

Damit sich die beiden Halteteile nicht in unerwünschter Weise aus der Verbindungsstellung lösen, ist es vorteilhaft, wenn ein Verriegelungsmechanismus vorgesehen ist zum Verriegeln der Verbindungsstellung der beiden Halteteile.

Der Aufbau des Sterilbehälters wird besonders einfach, wenn der Verriegelungsmechanismus eine Rastverbindung umfaßt. Diese hat je nach Ausführungsform auch den Vorteil, daß ein Verrasten der beiden Halteteile in der Verbindungsstellung akustisch wahrnehmbar sein kann.

Zum Schutz des Sterilfilters kann eine Abdeckung vorgesehen sein, welche das Sterilfilter einseitig überdeckt. Beispielsweise kann diese auf einer Innenseite oder einer Außenseite des Sterilbehälters angeordnet sein, je nachdem, ob das Sterilfilter auf einer Innen- oder Außenseite des Sterilbehälters angeordnet ist.

Um die Zahl der zur Herstellung des Sterilbehälters erforderlichen Teile zu minimieren, ist es günstig, wenn das zweite Halteteil die Abdeckung umfaßt.

Günstigerweise sind das zweite Halteteil und die Abdeckung einstückig ausgebildet. Auf diese Weise läßt sich auch eine Stabilität des zweiten Halteteils erhöhen.

Damit auf einfache Weise ein Gasaustausch durch die Abdeckung hindurch möglich ist, ist es günstig, wenn diese mit Durchbrechungen versehen ist zum Bereitstellen einer Fluidverbindung zwischen dem Aufnahmeraum und dem Sterilfilter.

Um das von der Abdeckung bedeckte Sterilfilter gegen mögliche Beschädigungen durch im Aufnahmeraum enthaltene Gegenstände zu schützen, ist es vorteilhaft, wenn die Durchbrechungen in einer Richtung quer zu einer Durchlaßrichtung mit Durchbrechungsabdeckungen bedeckt sind.

Ein Gasaustausch zwischen dem Aufnahmeraum und einer Umgebung des Sterilbehälters wird zusätzlich verbessert, wenn die Abdeckung vom Sterilfilter beabstandet ist und Stützelemente des ersten und/oder zweiten Halteteils Abstandshalter für die Abdeckung bilden.

Damit das Überdruckventil bei Druckdifferenzen zwischen dem Aufnahmeraum und der Umgebung des Sterilbehälters, die kleiner sind als die Mindestdruckdifferenz, zuverlässig die Grundstellung einnimmt, ist es günstig, wenn die Ventilklappe in der Grundstellung unter Vorspannung am Sterilbehälter gehalten ist.

Grundsätzlich könnte das Überdruckventil in Form eines Auslaßventils ausgebildet sein. Vorzugsweise ist das Überdruckventil jedoch in Form eines Einlaßventils ausgebildet, welches die Durchlaßstellung einnimmt, wenn ein in einer Umgebung des Sterilbehälters herrschender Druck einen im Aufnahmeraum herrschenden Druck um die Mindestdruckdifferenz übersteigt. Dadurch kann verhindert werden, das der Sterilbehälter zusammengedrückt wird, beispielsweise während eines Sterilisationsvorgangs. Eine Druckdifferenz kann dann abgebaut werden, indem unter hohem Druck stehender Heißdampf durch die Ventilöffnung in den Aufnahmeraum des Sterilbehälters einströmen kann.

Besonders einfach wird der Aufbau des Sterilbehälters, wenn das Sterilfilter die Ventilklappe bildet. Das Sterilfilter übt somit zwei Funktionen aus, einerseits dient es dazu, ein Eindringen von Keimen und Bakterien in den Aufnahmeraum zu verhindern, andererseits dient es als Ventilklappe der Ventilöffnung.

Damit die Ventilklappe nicht beschädigt werden kann, beispielsweise durch eine zu starke Verformung, ist es günstig, wenn mindestens ein Anschlag vorgesehen ist, welcher eine Öffnungsbewegung der Ventilklappe von der Ventilöffnung weg begrenzt.

Um einen Austausch des Sterilfilters möglichst selten vornehmen zu müssen, ist es vorteilhaft, wenn das Sterilfilter ein Dauerfilter ist, insbesondere ein aus Polytetrafluorethylen (PTFE) hergestelltes.

Besonders leicht wird der Sterilbehälter, wenn der Deckel aus einem Kunststoff hergestellt ist, insbesondere aus Polyetheretherketon (PEEK) oder Polyphenylensulfon (PPSU).

Die nachfolgende Beschreibung einer bevorzugten Ausführungsform der Erfindung dient im Zusammenhang mit der Zeichnung der näheren Erläuterung. Es zeigen:
- Figur 1:: eine Explosionsdarstellung eines Deckels eines Sterilbehälters;
- Figur 2 :: eine Draufsicht auf einen Ausschnitt einer Innenseite des Deckels mit einem Teil einer Filterhalterung;
- Figur 3 :: eine Schittansicht längs Linie 3-3 in Figur 2;
- Figur 4:: eine Schnittansicht ähnlich Figur 3 mit eingesetztem Sterilfilter;
- Figur 5:: eine vergrößerte Ansicht des Bereichs A in Figur 4;
- Figur 6:: eine Draufsicht auf einen Deckel der Filterhalterung;
- Figur 7:: eine Schittansicht längs Linie 7-7 in Figur 6;
- Figur 8:: eine Ansicht einer Unterseite des Deckels der Filterhalterung aus Figur 6; und
- Figur 9:: eine schematische Darstellung der Deckungsgleichheit von Stegen zweier Halteteile der Filterhalterung.

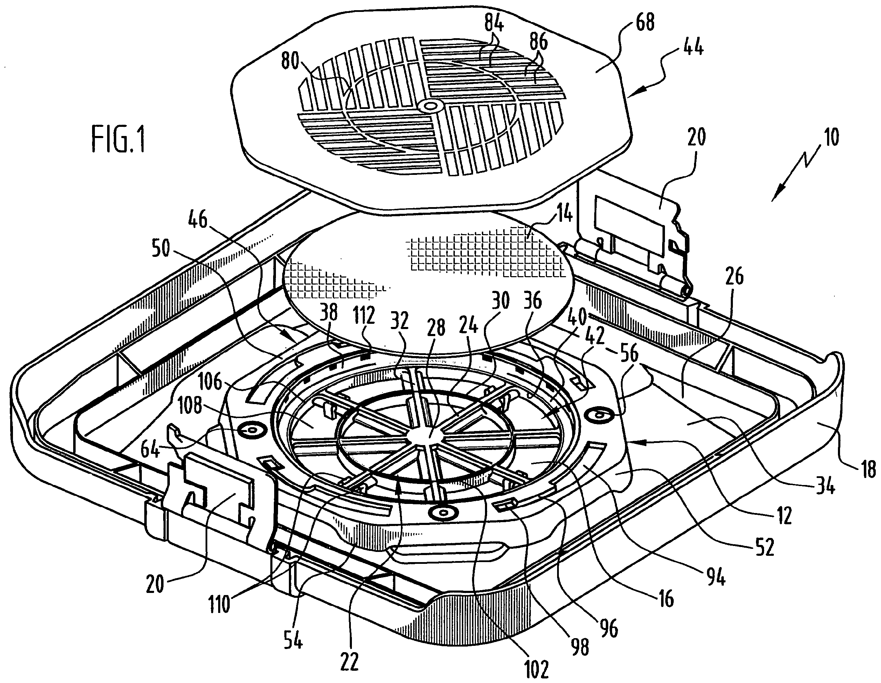

In Figur 1 ist ein insgesamt mit dem Bezugszeichen 10 versehener Deckel eines eine Behälterwanne umfassenden Sterilbehälters dargestellt. Am Deckel 10 ist eine Filterhalterung angeordnet und insgesamt mit dem Bezugszeichen 12 gekennzeichnet. Sie dient zur Aufnahme eines flachen, scheibenförmigen Sterilfilters 14, welches in einer Sterilstellung eine kreisförmige, zentral mittig am Deckel 10 angeordnete Öffnung 16 verschließt, um einerseits einen Gasaustausch zwischen einem von der Behälterwanne umschlossenen Innenraum und einer Umgebung des Sterilbehälters zu gestatten, andererseits ein Eindringen von Keimen und Bakterien in den Innenraum zu verhindern.

Der Deckel 10 ist in herkömmlicher Weise mit einem umlaufenden Rand 18 versehen, welcher Seitenwände der Behälterwanne teilweise überdeckt. Um den Deckel 10 mit der Behälterwanne zu verbinden und an dieser zu sichern, sind am Rand 18 des Deckels 10 verschwenkbar gelagerte Schließbügel 20 vorgesehen.

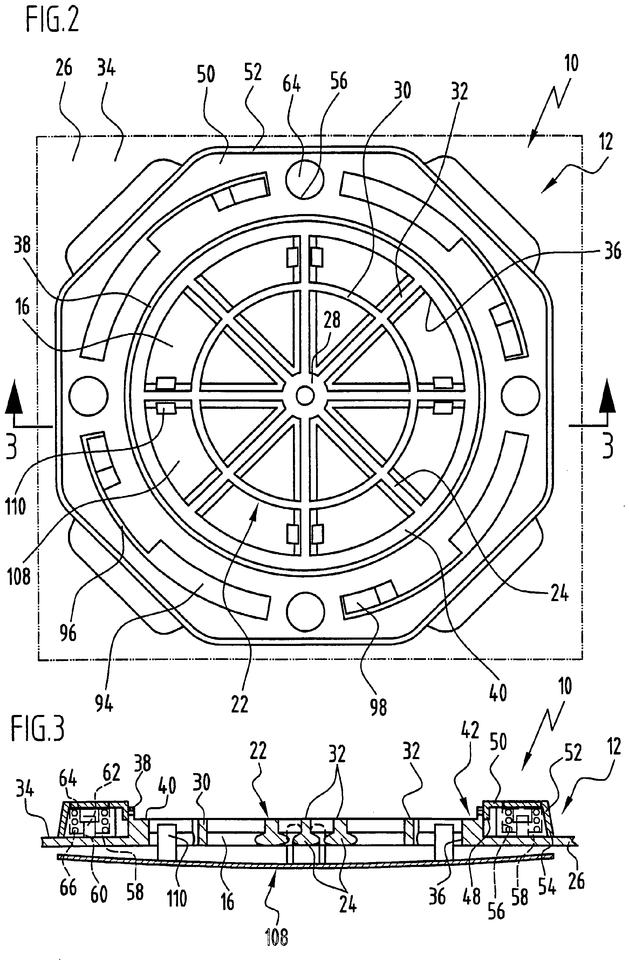

Die kreisrunde Öffnung 16 wird durch eine insgesamt mit dem Bezugszeichen 22 versehene, in den Figuren 2 bis 5 dargestellte Stützstruktur in einzelne Segmente unterteilt. Die Stützstruktur 22 ist symmetrisch ausgebildet und umfaßt acht, von einem Zentrum der Öffnung 16 ausgehende radiale Stützstege 24, welche einstückig mit einer Wand 26 des Deckels 10 verbunden sind. Eine im Zentrum der Öffnung 16 angeordnete kleine Stützscheibe 28 ist konzentrisch von einem Stützring 30 umgeben, welcher die Stützstege 24 in etwa auf der Hälfte ihrer Gesamtlänge kreuzt. Die Stützstruktur 22 ist so ausgebildet, das deren Teilelemente insgesamt eine ebene Auflagefläche 32 bilden, welche eine von der Wand 26 des Deckels 10 definierte Innenfläche 34 über die Öffnung 16 hinweg zumindest abschnittsweise fortsetzt. Beabstandet von einem Rand 36 der Öffnung 16 ist ein senkrecht von der Innenfläche 34 abstehender, ringförmiger Führungsrand 38 vorgesehen, so daß eine ringförmige, die Öffnung 16 umgebende Stützfläche 40 durch die Innenfläche 34 gebildet wird. Der Führungsrand 38 zusammen mit der Stützstruktur 22 bildet eine topfförmige Filteraufnahme 42, wobei ein Durchmesser des Sterilfilters 14 zu einem Innendurchmesser des ringförmigen Führungsrands 38 korrespondiert, so daß das in die Filteraufnahme 42 eingelegte Sterilfilter 14 seitlich geführt ist.

Die Filteraufnahme 42 bildet einen Teil der Filterhalterung 12, welche ferner einen Klemmdeckel 44 sowie einen beweglich an der Wand 26 gelagerten und mit dem Klemmdeckel 44 lösbar verbindbaren Rahmen 46 umfaßt. Der Rahmen 46 ist im Querschnitt in etwa U-förmig ausgebildet und weist eine am Führungsrand 38 anliegende ringförmige Seitenwand 48 sowie eine parallel zur Innenfläche 34 verlaufende Stützwand 50 auf. An der Stützwand 50 ist eine im Wesentlichen quer abstehende Außenwand 52 angeordnet, deren Rand 54 an der Innenfläche 34 des Deckels 10 anliegt. In einer Draufsicht bildet der Rand 54 eine achteckige Begrenzung des Rahmens 46.

An der Stützwand 50 senkrecht abstehend sind symmetrisch über den Umfang des Rahmens 46 verteilt vier hohlzylindrische Lagertöpfe 56 angeordnet, deren Boden 58 an der Innenfläche 34 anliegt und eine zentrale Bohrung 60 aufweist, durch welche ein senkrecht von der Innenfläche 34 abstehender Lagerbolzen 62 hindurchragt. Auf diesen ist ein Topfdeckel 64 aufgesetzt, welcher den Lagertopf 56 verschließt und flächig in die Stützwand 50 eingepaßt ist. Im Lagertopf 56 stützt sich einerseits am Topfdeckel 64, andererseits am Boden 58 eine Schraubenfeder 66 ab. Wird der Rahmen 46 parallel zu einer Längsachse der Lagerbolzen 62 von der Innenfläche 34 weg bewegt, so wird die Schraubenfeder 66 zusammengedrückt. Eine Bewegung des Rahmens 46 relativ zur Wand 26 des Deckels 10 wird durch den an einen Innendurchmesser des Lagertopfs 56 angepaßten Topfdeckel 64 sowie die Bohrung 60 im Boden 58 und den Lagerbolzen 62 geführt. Ferner wird eine seitliche Bewegung des Rahmens 46 relativ zur Filteraufnahme 42 durch die an den Führungsrand 38 im Durchmesser angepaßte Seitenwand 48 unterbunden.

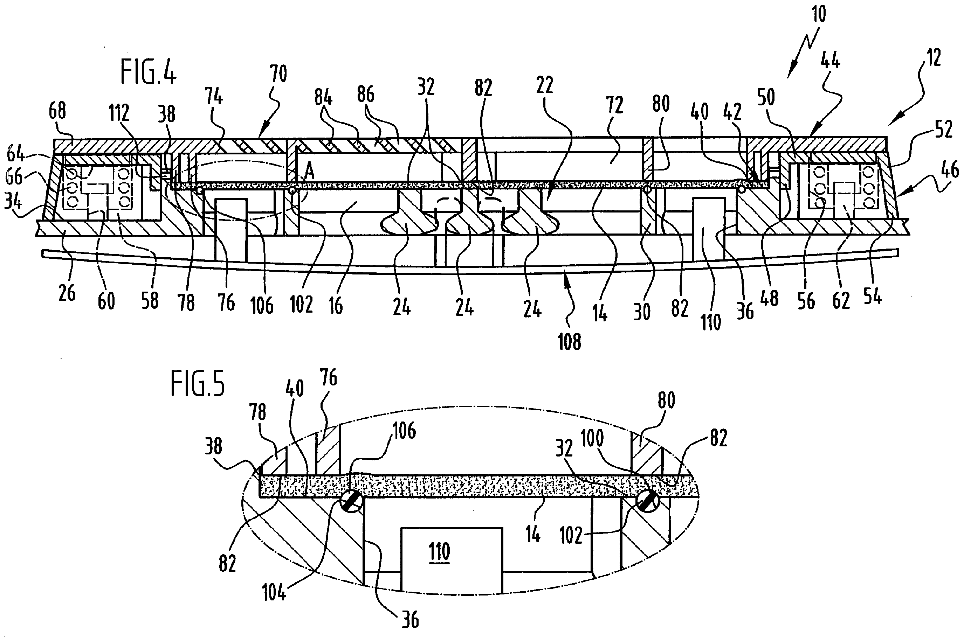

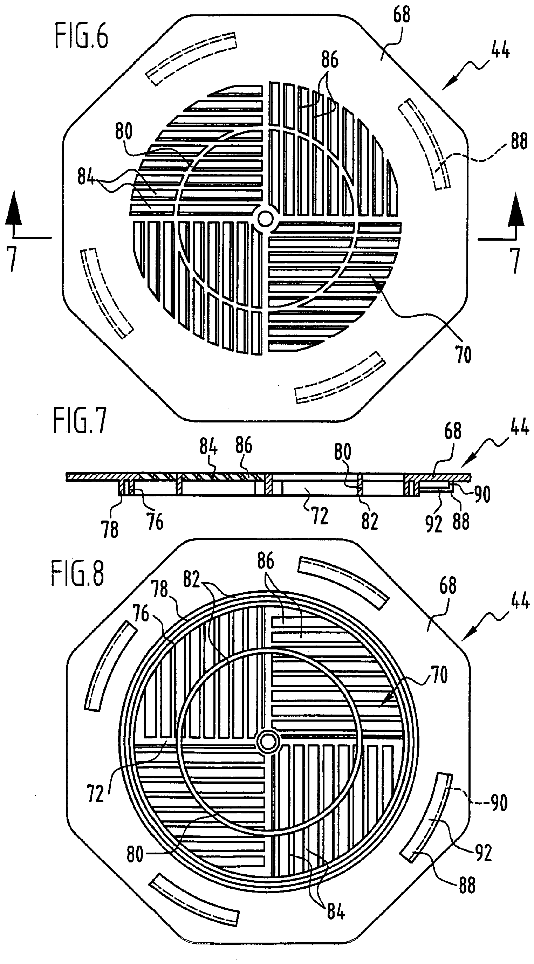

Der in den Figuren 6 bis 8 näher dargestellte Klemmdeckel 44 umfaßt eine flache, achteckige Platte 68, welche in einer Verbindungsstellung, wie sie in Figur 4 dargestellt ist, die Stützwand 50 des achteckigen Rahmens 46 gerade bedeckt. Die Platte 68 ist mit einer kreisförmigen Öffnung 70 versehen, welche von vier Stützstegen 72 in vier Viertelkreissegmente unterteilt ist. Benachbart einem inneren Rand 74 der Öffnung 70 sind konzentrisch und parallel zueinander zwei Klemmringe 76 und 78 senkrecht aufstehend an der Platte 68 angeordnet. Konzentrisch zu diesen ist ferner ein ein Zentrum der Öffnung 70 umgebender Klemmring 80 angeordnet, welcher die Stützstege 72 kreuzt. Die Stützstege 72 sowie die Klemmringe 76, 78 und 80 definieren gemeinsam eine ebene Klemmfläche 82 für das Sterilfilter 14. Ein Außendurchmesser des Klemmrings 78 ist so gewählt, das der Klemmring 78 außen am Führungsrand 38 anliegt. Der Führungsrand 38 wird somit zwischen der Seitenwand 48 und dem Klemmring 78 geführt. Die Öffnung 70 zusätzlich bedeckend sind eine Vielzahl von Lamellen 84 vorgesehen, wobei die Lamellen 84 jedes von den Stützstegen 72 definierten Sektors der Öffnung 70 parallel zueinander ausgerichtet sind, die Lamellen 84 benachbarter Sektoren jedoch quer zueinander. Jede der Lamellen 84 ist relativ zur Platte 68 um 45° geneigt, so daß schräge Durchtrittsschlitze 86 entstehen, durch welche ein Gasaustausch von einer Seite der Platte 68 zur anderen Seite derselben ermöglicht wird.

Zum Verbinden des Klemmdeckels 44 mit dem Rahmen 46 sind vier konzentrisch zu den Klemmringen 76 und 78 von der Platte 68 abstehende Vorsprünge 88 angeordnet, die sich jeweils über einen Winkelbereich von etwa 30° erstrecken und im Querschnitt L- förmig ausgebildet sind. Sie weisen einen senkrecht von der Platte 68 abstehenden Anschnitt 90 sowie einen parallel zur Platte 68 vom Abschnitt 90 abstehenden, auf die Klemmringe 76 und 78 hin weisenden Halteabschnitt 92 auf.

Die Vorsprünge 88 bilden einen Teil einer Bajonettverbindung zum Verbinden des Klemmdeckels 44 mit dem Rahmen 46. Diese umfaßt ferner für jeden Vorsprung 88 einen Bajonettschlitz 94, in welchen der Halteabschnitt 92 eingesetzt werden kann. An den Bajonettschlitz 94 schließt sich ein schmalerer, zum Abschnitt 90 korrespondierender Führungsschlitz 96 an, so daß bei Verdrehung des mit den Vorsprüngen 88 in den Bajonettschlitz 94 eingesetzten Klemmdeckels 44 bei an der Stützwand 50 anliegender Platte 68 eine Verdrehung des Klemmdeckels 44 relativ zum Rahmen 46 geführt wird, und zwar durch die in die Führungsschlitze 96 eintauchenden Abschnitte 90. Um den Klemmdeckel 44 in der Verbindungsstellung zu halten, sind benachbart den Führungsschlitzen 96 angeordnete Federzungen 98 vorgesehen, welche in der Verbindungsstellung gegen die Abschnitte 90 drücken. Zusätzlich sind an den Federzungen 98 nicht näher dargestellte Rastvorsprünge angeordnet, die in der Verbindungsstellung eine Schmalseite des Abschnitts 90 hintergreifen und eine Drehbewegung aus der Verbindungsstellung heraus verhindern.

Um eine besonders gute Abdichtung der Öffnung 16 durch den Sterilfilter 14 zu erreichen, ist im Stützring 30 eine Ringnut 100 angeordnet, in weicher ein Dichtring 102 eingesetzt ist. Ebenso ist die Dichtfläche 40 mit einer Ringnut 104 versehen, welche ebenfalls einen Dichtring 106 aufnimmt. In der Steril- oder Schließstellung, die in den Figuren 4 und 5 dargestellt ist, liegt das Sterilfilter 14 an den Dichtringen 102 und 106 an und verschließt die Öffnung 16 vollständig.

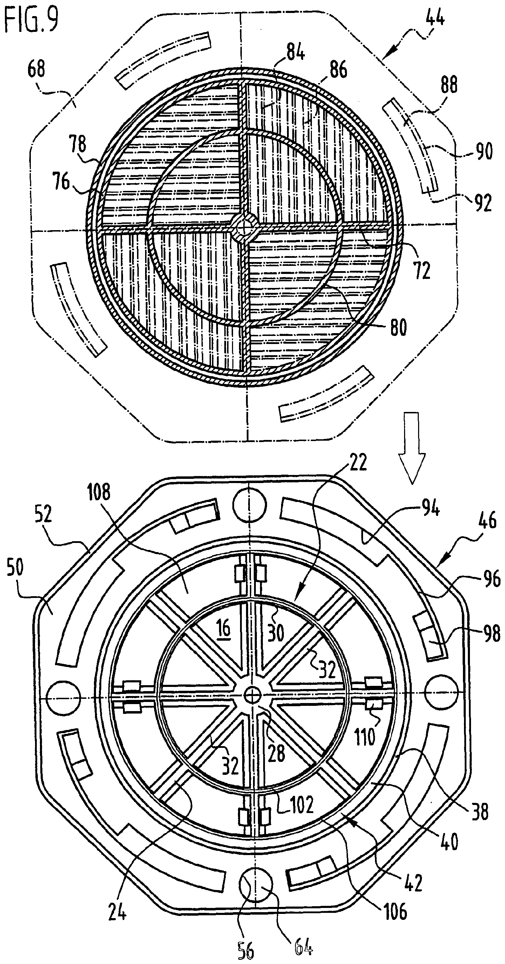

Die Durchmesser des Stützrings 30 und des Klemmrings 80 entsprechen einander, so daß die vom Stützring 30 gebildete Auflagefläche 32 sowie die vom Klemmring 80 gebildete Klemmfläche 82 einander direkt gegenüberliegend angeordnet sind und aneinander anliegen würden, wenn kein Sterilfilter 14 zwischen ihnen klemmend gehalten wäre. Ebenso liegt die Stützfläche 40 der von den beiden Klemmringen 76 und 80 gebildeten Klemmfläche 82 gegenüber, so daß ein äußerer Rand des Sterilfilters 14 klemmend gehalten werden kann. In Figur 9 ist die konzentrische und gegenüberliegende beziehungsweise überdeckende Ausgestaltung der einander gegenüberliegenden Stützfläche 40 beziehungsweise Klemmfläche 82 dargestellt, wenn nämlich der Klemmdeckel 44 eine den Rahmen 46 überdeckende Stellung einnimmt.

Die Stützstege 72 des Klemmdeckels 44 sind so angeordnet, daß sie in der Verbindungsstellung, das heißt nach Einsetzen der Vorsprünge 88 in die Bajonettschlitze 94 und Verdrehen des Klemmdeckels 44 um 45° relativ zum Rahmen 46 über jeweils einem Stützsteg 24 angeordnet sind, so daß der Sterilfilter 14 zwischen von den Stützstegen 72 und 24 gebildeten Klemmflächen 82 beziehungsweise Auflageflächen 32 klemmend gehalten ist.

Um das Sterilfilter 14 gegen eine Beschädigung von außen zu schützen, ist ein die Öffnung 16 überdeckender, schwach von der Wand 26 weg gewölbter Schutzdeckel 108 vorgesehen, welcher vier Verbindungsclips 110 aufweist, welche an die Stützstege 24 anclipbar sind. Der Schutzdeckel 108 läßt sich dadurch auf einfache Weise am Deckel 10 durch anclipsen befestigen.

Die Filterhalterung 12 ist insgesamt so ausgebildet, daß sie zusammen mit dem in ihr gehaltenen Sterilfilter 14 ein Überdruckventil bildet. Übersteigt nämlich ein in der Umgebung der Sterilbehälters herrschender Druck den innerhalb des Sterilbehälters, so wird das Sterilfilter 14 infolge der von außen auf es einwirkenden Druckkräfte gegen den Klemmdeckel 44 gedrückt. Da dieser fest mit dem Rahmen 46 verbunden ist, der Rahmen 46 jedoch beweglich am Deckel 10 gelagert ist, wird dieser ebenfalls von der Wand 26 weg bewegt, so daß sich die Böden 58 von der Innenfläche 34 lösen und die Schraubenfedern 66 zusammengedrückt werden. Dies ermöglicht einen Gasaustausch zwischen der Umgebung und dem Innenraum des Sterilbehälters nicht nur durch das Sterilfilter 14 hindurch, sondern auch durch einen sich öffnenden Überdruckströhmungspfad zwischen der Stützfläche 40 und dem Sterilfilter 14. Das Überdruckventil beziehungsweise das Sterilfilter nehmen dann die Durchlaßstellung ein.

Um einen möglichst ungehinderten Gasaustausch zu gestatten, ist der Führungsrand 38 mit parallel zur Innenfläche 34 verlaufenden Schlitzen 112 versehen, die regelmäßig über den Umfang des Führungsrands 38 verteilt sind. Sie werden freigegeben, wenn der Klemmdeckel 44 die beschriebene Durchlaßstellung einnimmt. Verringert sich die Druckdifferenz zwischen der Umgebung und dem Innenraum des Sterilbehälters wieder, dann wird der Rahmen und damit der mit diesem verbundene Klemmdeckel 44 durch die Schraubenfedern 66 wieder in die in Figur 4 dargestellte Sterilstellung überführt, in welcher das Sterilfilter 14, welches eine Ventilklappe des Überdruckventils bildet, die Öffnung 16 vollständig verschließt.

Bis auf die Schließbügel 20 ist der gesamte Deckel 10 bei dem beschriebenen Ausführungsbeispiel aus Kunststoff hergestellt. Allerdings wäre es denkbar, die Schließbügel 20 auch aus einem Kunststoff herzustellen, so daß der gesamte Deckel mit allen daran angeordneten Teilen aus einem Kunststoff hergestellt sein kann. Bei anderen Deckelvarianten können jedoch auch unterschiedliche Materialien zur Herstellung des Deckels 10 verwendet werden, insbesondere für Teile des Deckels 10, die nicht einstückig miteinander ausgebildet sind und zur Bereitstellung besonderer Funktionalitäten dienen. Beim oben beschriebenen Ausführungsbeispiel sind der Klemmdeckel 44, der Rahmen 46, der Schutzdeckel 108 und der Deckel 10 jeweils einstückig aus einem Kunststoff hergestellt.

## Patentansprüche

1. Sterilbehälter, insbesondere zur Aufnahme und sterilen Aufbewahrung von chirurgischem Besteck oder chirurgischem Material, mit einem durch einen Behälterboden und Behälterwände gebildeten Aufnahmeraum, mit einem Deckel (10) zum Verschließen des Aufnahmeraums, mit einer Gasaustauschöffnung (16) zum Bereitstellen einer Fluidverbindung zwischen dem Aufnahmeraum und einer Umgebung des Sterilbehälters, mit einer ein erstes und ein zweites Halteteil (22, 44) umfassenden Filterhalterung (12) und mit einem Sterilfilter (14), wobei das Sterilfilter (14) in einer Sterilstellung, in welcher das Sterilfilter (14) die Gasaustauschöffnung (16) verschließt, zwischen dem ersten und dem zweiten Halteteil (22, 44, 46) gehalten ist, wobei das Sterilfilter (14) durchbrechungsfrei ausgebildet ist, wobei das erste Halteteil (22) mindestens eine erste Haltefläche (32) aufweist, wobei das zweite Halteteil (44) mindestens eine zweite Haltefläche (82) aufweist und wobei das Sterilfilter (14) zwischen der ersten und der zweiten Haltefläche (32, 82) in der Sterilstellung klemmend gehalten ist, wobei mindestens ein Überdruckventil (14, 16, 44, 46) zum Bereitstellen einer Fluidverbindung zwischen einer Umgebung und dem Aufnahmeraum des Sterilbehälters vorgesehen ist und das Überdruckventil (14, 16, 44, 46) eine Ventilöffnung (16) und eine Ventilklappe (14) zum Öffnen und Schließen der Ventilöffnung (16) umfaßt, wobei das Überdruckventil (14, 16, 44, 46) derart ausgebildet ist, daß in einer Grundstellung die Ventilöffnung (16) geschlossen ist und daß in einer Durchlaßstellung bei Überschreiten einer Mindestdruckdifferenz zwischen im Aufnahmeraum und in der Umgebung des Sterilbehälters herrschenden Drücken die Ventilöffnung (16) mindestens teilweise geöffnet ist, **dadurch gekennzeichnet, daß** das erste Halteteil (22), das zweite Halteteil (46) und das Sterilfilter (14) miteinander lösbar verbindbar sind, daß eine Wandfläche (34) des Sterilbehälters einstückig mit der mindestens einen ersten Haltefläche (32) ausgebildet ist, daß das zweite Halteteil (46) relativ zum ersten Halteteil (22, 44) beweglich gelagert ist, daß Lagerungselemente (56, 62) am zweiten Halteteil (46) und am Sterilbehälter benachbart der Gasaustauschöffnung zur beweglichen Lagerung des zweiten Halteteils (46) am Sterilbehälter vorgesehen sind und daß das Sterilfilter (14) in der Grundstellung zwischen der mindestens einen ersten und der mindestens einen zweiten Haltefläche (32, 82) unter Vorspannung gehalten ist.

2. Sterilbehälter nach Anspruch 1, **dadurch gekennzeichnet, daß** die mindestens eine erste und die mindestens eine zweite Haltefläche (32, 82) einander gegenüberliegend angeordnet sind.

3. Sterilbehälter nach einem der voranstehenden Ansprüche, **dadurch gekennzeichnet, daß** das Sterilfilter (14) und/oder die Gasaustauschöffnung (16) kreisförmig oder im Wesentlichen kreisförmig ausgebildet sind.

4. Sterilbehälter nach einem der voranstehenden Ansprüche, **dadurch gekennzeichnet, daß** die Gasaustauschöffnung (16) am Deckel (10) vorgesehen ist und daß die Wandfläche (34) einen Teil des Deckels (10) des Sterilbehälters bildet.

5. Sterilbehälter nach einem der voranstehenden Ansprüche, **dadurch gekennzeichnet, daß** das erste Halteteil (22) eine Sterilfilteraufnahme (42) umfaßt, welche eine Querschnittsfläche aufweist, die im wesentlichen zu einer Filterfläche des Sterilfilters (14) korrespondiert.

6. Sterilbehälter nach Anspruch 5, **dadurch gekennzeichnet, daß** die Sterilfilteraufnahme (42) topfartig ausgebildet ist.

7. Sterilbehälter nach einem der Ansprüche 5 oder 6, **dadurch gekennzeichnet, daß** in einer Seitenwand (38) der Sterilfilteraufnahme (42) Durchbrechungen (112) vorgesehen sind.

8. Sterilbehälter nach einem der voranstehenden Ansprüche, **dadurch gekennzeichnet, daß** mindestens ein Stützelement (24, 30; 72, 76, 78, 80) vorgesehen ist, welches die mindestens eine erste Haltefläche (32) oder die mindestens eine zweite Haltefläche (82) umfaßt und welches die Gasaustauschöffnung (16) mindestens teilweise überdeckende Auflageflächen (32, 82) zum einseitigen Stützen des Sterilfilters (14) umfaßt.

9. Sterilbehälter nach Anspruch 8, **dadurch gekennzeichnet, daß** das mindestens eine Stützelement (24, 30; 72, 76, 78, 80) ein von einem Zentrum der Gasaustauschöffnung (16) ausgehender radialer Stützsteg (24; 72) ist.

10. Sterilbehälter nach Anspruch 8 oder 9, **dadurch gekennzeichnet, daß** das mindestens eine erste Stützelement (24, 30; 72, 76, 78, 80) ein zur Gasaustauschöffnung (16) konzentrischer Stützring (30; 76, 78, 80) ist.

11. Sterilbehälter nach Anspruch 10, **dadurch gekennzeichnet, daß** zwei oder drei konzentrische Stützringe (76, 78, 80) vorgesehen sind.

12. Sterilbehälter nach einem der voranstehenden Ansprüche, **dadurch gekennzeichnet, daß** das mindestens eine erste und/oder das mindestens eine zweite Halteteil (22, 44, 46) eine Dichtung (102, 106) tragen, an welcher das Sterilfilter (14) in der Sterilstellung anliegt.

13. Sterilbehälter nach Anspruch 12, **dadurch gekennzeichnet, daß** das mindestens eine erste und/oder das mindestens eine zweite Halteteil (22, 44, 46) eine Dichtungsaufnahme (100, 104) aufweisen, in welche die Dichtung (102, 106) mindestens teilweise eintaucht.

14. Sterilbehälter nach einem der Ansprüche 12 oder 13, **dadurch gekennzeichnet, daß** die Dichtung (102, 106) in Form eines Dichtringes (102, 106) ausgebildet ist.

15. Sterilbehälter nach einem der voranstehenden Ansprüche, **dadurch gekennzeichnet, daß** das erste Halteteil (22, 44) und das zweite Halteteil (46) miteinander lösbar verbindbar sind.

16. Sterilbehälter nach einem der voranstehenden Ansprüche, **dadurch gekennzeichnet, daß** das Sterilfilter (14) und die Filterhalterung (12) lösbar verbindbar sind und daß das Sterilfilter (14) in einer Entnahmestellung von der Filterhalterung (12) lösbar und in einer Verbindungsstellung an der Filterhalterung (12) gehalten ist.

17. Sterilbehälter nach Anspruch 16, **dadurch gekennzeichnet, daß** eine Bajonettverbindung (88, 94, 96) zum Verbinden des ersten Halteteils (22, 44) mit dem zweiten Halteteil (46) und zum Überführen der Filterhalterung (12) von der Entnahmestellung in die Verbindungsstellung vorgesehen ist.

18. Sterilbehälter nach einem der Ansprüche 16 oder 17, **dadurch gekennzeichnet, daß** ein Verriegelungsmechanismus (98) vorgesehen ist zum Verriegeln der Verbindungsstellung der beiden Halteteile (22, 44, 46).

19. Sterilbehälter nach Anspruch 18, **dadurch gekennzeichnet, daß** der Verriegelungsmechanismus (98) eine Rastverbindung umfaßt.

20. Sterilbehälter nach einem der voranstehenden Ansprüche, **dadurch gekennzeichnet, daß** eine Abdeckung (68; 108) vorgesehen ist, welche das Sterilfilter (14) einseitig überdeckt.

21. Sterilbehälter nach Anspruch 20, **dadurch gekennzeichnet, daß** das zweite Halteteil (46) die Abdeckung (68) umfaßt.

22. Sterilbehälter nach Anspruch 20 oder 21, **dadurch gekennzeichnet, daß** das zweite Halteteil (46) und die Abdeckung (68) einstückig ausgebildet sind.

23. Sterilbehälter nach einem der Ansprüche 20 bis 22, **dadurch gekennzeichnet, daß** die Abdeckung (68) mit Durchbrechungen (86) versehen ist zum Bereitstellen einer Fluidverbindung zwischen dem Aufnahmeraum und dem Sterilfilter (14).

24. Sterilbehälter nach Anspruch 23, **dadurch gekennzeichnet, daß** die Durchbrechungen (86) in einer Richtung quer zu einer Durchlaßrichtung mit Durchbrechungsabdeckungen (84) bedeckt sind.

25. Sterilbehälter nach einem der Ansprüche 20 bis 24, **dadurch gekennzeichnet, daß** die Abdeckung (68) vom Sterilfilter (14) beabstandet ist und daß Stützelemente (72, 76, 78, 80) des ersten und/oder zweiten Halteteils (22, 44, 46) Abstandshalter (72, 76, 78, 80) für die Abdeckung (68) bilden.

26. Sterilbehälter nach einem der voranstehenden Ansprüche, **dadurch gekennzeichnet, daß** die Ventilklappe (14) in der Grundstellung unter Vorspannung am Sterilbehälter gehalten ist.

27. Sterilbehälter nach einem der voranstehenden Ansprüche , **dadurch gekennzeichnet, daß** das Überdruckventil (14, 16, 44, 46) in Form eines Einlaßventils ausgebildet ist, welches die Durchlaßstellung einnimmt, wenn ein in einer Umgebung des Sterilbehälters herrschender Druck einen im Aufnahmeraum herrschenden Druck um die Mindestdruckdifferenz übersteigt.

28. Sterilbehälter nach einem der voranstehenden Ansprüche , **dadurch gekennzeichnet, daß** das Sterilfilter (14) die Ventilklappe (14) bildet.

29. Sterilbehälter nach einem der voranstehenden Ansprüche , **dadurch gekennzeichnet, daß** mindestens ein Anschlag (58, 64) vorgesehen ist, welcher eine Öffnungsbewegung der Ventilklappe (14) von der Ventilöffnung (16) weg begrenzt.

30. Sterilbehälter nach einem der voranstehenden Ansprüche, **dadurch gekennzeichnet, daß** das Sterilfilter (14) ein Dauerfilter ist, insbesondere ein aus Polytetrafluorethylen (PTFE) hergestelltes.

31. Sterilbehälter nach einem der voranstehenden Ansprüche, **dadurch gekennzeichnet, daß** der Deckel (10) aus einem Kunststoff hergestellt ist, insbesondere aus Polyetheretherketon (PEEK) oder Polyphenylensulfon (PPSU).

## Claims

1. Sterile container, in particular, for receiving and storing surgical instruments or surgical material under sterile conditions, comprising a receiving space formed by a container bottom and container walls, a lid (10) for closing the receiving space, a gas exchange opening (16) for providing a fluid connection between the receiving space and an environment outside of the sterile container, a filter holding device (12) including a first and a second holding element (22, 44), and a sterile filter (14), the sterile filter (14) being held between the first and the second holding elements (22, 44, 46) in a sterile position in which the sterile filter (14) closes the gas exchange opening (16), the sterile filter (14) being constructed so as to have no openings, the first holding element (22) having at least one first holding surface (32), the second holding element (44) having at least one second holding surface (82), and the sterile filter (14) being held clamped between the first and the second holding surfaces (32, 82) in the sterile position, at least one pressure-relief valve (14, 16, 44, 46) being provided for providing a fluid connection between an outside environment and the receiving space of the sterile container, and the pressure-relief valve (14, 16, 44, 46) comprising a valve opening (16), and a valve flap (14) for opening and closing the valve opening (16), the pressure-relief valve (14, 16, 44, 46) being constructed such that in a normal position the valve opening (16) is closed, and that in a flow-through position, when a minimum pressure difference between pressures prevailing in the receiving space and in the outside environment of the sterile container is exceeded, the valve opening (16) is at least partially open, **characterized in that** the first holding element (22), the second holding element (46) and the sterile filter (14) are releasably connectable to one another, **in that** a wall surface (34) of the sterile container is formed integrally with the at least one first holding surface (32), **in that** the second holding element (46) is mounted so as to be movable relative to the first holding element (22, 44), **in that** bearing elements (56, 62) are provided on the second holding element (46) and on the sterile container adjacent to the gas exchange opening for mounting the second holding element (46) movably on the sterile container, and **in that** in the normal position the sterile filter (14) is held in a biased manner between the at least one first and the at least one second holding surfaces (32, 82).

2. Sterile container in accordance with claim 1, **characterized in that** the at least one first and the at least one second holding surfaces (32, 82) are arranged so as to face one another.

3. Sterile container in accordance with any one of the preceding claims, **characterized in that** the sterile filter (14) and/or the gas exchange opening (16) are of circular or substantially circular construction.

4. Sterile container in accordance with any one of the preceding claims, **characterized in that** the gas exchange opening (16) is provided on the lid (10), and **in that** the wall surface (34) forms part of the lid (10) of the sterile container.

5. Sterile container in accordance with any one of the preceding claims, **characterized in that** the first holding element (22) includes a sterile filter receptacle (42) having a cross sectional area that corresponds substantially to a filter surface of the sterile filter (14).

6. Sterile container in accordance with claim 5, **characterized in that** the sterile filter receptacle (42) is of pot-like construction.

7. Sterile container in accordance with claim 5 or 6, **characterized in that** openings (112) are provided in a side wall (38) of the sterile filter receptacle (42).

8. Sterile container in accordance with any one of the preceding claims, **characterized in that** at least one supporting element (24, 30; 72, 76, 78, 80) is provided, which includes the at least one first holding surface (32) or the at least one second holding surface (82), and which includes bearing surfaces (32, 82) covering at least partially the gas exchange opening (16) for supporting the sterile filter (14) on one side thereof.

9. Sterile container in accordance with claim 8, **characterized in that** the at least one supporting element (24, 30; 72, 76, 78, 80) is a radial supporting web (24; 72) extending from a center of the gas exchange opening (16).

10. Sterile container in accordance with claim 8 or 9, **characterized in that** the at least one first supporting element (24, 30; 72, 76, 78, 80) is a supporting ring (30; 76, 78, 80) which is concentric in relation to the gas exchange opening (16).

11. Sterile container in accordance with claim 10, **characterized in that** two or three concentric supporting rings (76, 78, 80) are provided.

12. Sterile container in accordance with any one of the preceding claims, **characterized in that** the at least one first and/or the at least one second holding element (22, 44, 46) carry a seal (102, 106) on which the sterile filter (14) rests in the sterile position.

13. Sterile container in accordance with claim 12, **characterized in that** the at least one first and/or the at least one second holding element (22, 44, 46) have a seal receptacle (100, 104) in which the seal (102, 106) is seated at least partially.

14. Sterile container in accordance with claim 12 or 13, **characterized in that** the seal (102, 106) is constructed in the form of a sealing ring (102, 106).

15. Sterile container in accordance with any one of the preceding claims, **characterized in that** the first holding element (22, 44) and the second holding element (46) are releasably connectable to one another.

16. Sterile container in accordance with any one of the preceding claims, **characterized in that** the sterile filter (14) and the filter holding device (12) are releasably connectable, and **in that** the sterile filter (14) is releasable from the filter holding device (12) in a remove position and is held on the filter holding device (12) in a connect position.

17. Sterile container in accordance with claim 16, **characterized in that** a bayonet connector (88, 94, 96) is provided for connecting the first holding element (22, 44) to the second holding element (46) and for transferring the filter holding device (12) from the remove position to the connect position.

18. Sterile container in accordance with claim 16 or 17, **characterized in that** a locking mechanism (98) is provided for locking the connect position of the two holding elements (22, 44, 46).

19. Sterile container in accordance with claim 18, **characterized in that** the locking mechanism (98) comprises a snap-in locking connection.

20. Sterile container in accordance with any one of the preceding claims, **characterized in that** a cover (68; 108) is provided for covering the sterile filter (14) on one side thereof.

21. Sterile container in accordance with claim 20, **characterized in that** the second holding element (46) includes the cover (68).

22. Sterile container in accordance with claim 20 or 21, **characterized in that** the second holding element (46) and the cover (68) are of integral construction.

23. Sterile container in accordance with any one of claims 20 to 22, **characterized in that** the cover (68) is provided with openings (86) for providing a fluid connection between the receiving space and the sterile filter (14).

24. Sterile container in accordance with claim 23, **characterized in that** the openings (86) are covered by opening covers (84) in a direction transverse to a flow-through direction.

25. Sterile container in accordance with any one of claims 20 to 24, **characterized in that** the cover (68) is spaced from the sterile filter (14), and **in that** supporting elements (72, 76, 78, 80) of the first and/or the second holding elements (22, 44, 46) form spacers (72, 76, 78, 80) for the cover (68).

26. Sterile container in accordance with any one of the preceding claims, **characterized in that** in the normal position the valve flap (14) is held in a biased manner on the sterile container.

27. Sterile container in accordance with any one of the preceding claims, **characterized in that** the pressure-relief valve (14, 16, 44, 46) is constructed in the form of an inlet valve which assumes the flow-through position when a pressure prevailing in an environment outside of the sterile container exceeds a pressure prevailing in the receiving space by the minimum pressure difference.

28. Sterile container in accordance with any one of the preceding claims, **characterized in that** the sterile filter (14) forms the valve flap (14).

29. Sterile container in accordance with any one of the preceding claims, **characterized in that** at least one stop (58, 64) is provided for delimiting an opening movement of the valve flap (14) away from the valve opening (16).

30. Sterile container in accordance with any one of the preceding claims, **characterized in that** the sterile filter (14) is a permanent filter, in particular, one made from polytetrafluoroethylene (PTFE).

31. Sterile container in accordance with any one of the preceding claims, **characterized in that** the lid (10) is made from a plastic material, in particular, from polyetheretherketone (PEEK) or polyphenylene sulfone (PPSU).

## Revendications

1. Contenant ou récipient stérile, notamment destiné à accueillir et à assurer une conservation stérile d'instruments chirurgicaux ou de matériel chirurgical, comprenant
un compartiment d'accueil formé par un fond de contenant et des parois de contenant,
un couvercle (10) destiné à fermer le compartiment d'accueil,
une ouverture d'échange de gaz (16) pour réaliser une liaison fluidique entre le compartiment d'accueil et un environnement du contenant stérile,
un système de maintien de filtre (12) comportant une première et une deuxième pièce de maintien (22, 44), et un filtre stérile (14),
contenant
dans lequel le filtre stérile (14), dans une position stérile dans laquelle le filtre stérile (14) ferme l'ouverture d'échange de gaz (16), est maintenu entre la première et la deuxième pièce de maintien (22, 44, 46), dans lequel le filtre stérile (14) est d'une réalisation sans ouverture de passage,
dans lequel la première pièce de maintien (22) présente au moins une première surface de maintien (32),
dans lequel la deuxième pièce de maintien (44) présente au moins une deuxième surface de maintien (82),
et dans lequel le filtre stérile (14) est maintenu serré entre la première et la deuxième surface de maintien (32, 82), dans la position stérile,
dans lequel il est prévu au moins une valve de sureté de pression (14, 16, 44, 46) pour établir une liaison fluidique entre un environnement et le compartiment d'accueil du contenant stérile, et la valve de sureté de pression (14, 16, 44, 46) comprend une ouverture de valve (16) et un clapet de valve (14) pour ouvrir et fermer l'ouverture de valve (16),
dans lequel la valve de sureté de pression (14, 16, 44, 46) est configurée de manière telle, que dans une position de base, l'ouverture de valve (16) soit fermée, et que dans une position de passage, lors du dépassement d'une différence de pression minimum entre des pressions régnant dans le compartiment d'accueil et dans l'environnement du contenant stérile, l'ouverture de valve (16) soit au moins partiellement ouverte, **caractérisé en ce que** la première pièce de maintien (22), la deuxième pièce de maintien (46) et le filtre stérile (14) peuvent être reliés mutuellement de manière amovible, **en ce qu'**une surface de paroi (34) du contenant stérile est formée d'un seul tenant avec ladite au moins une première surface de maintien (32), **en ce que** la deuxième pièce de maintien (46) est montée de manière à être mobile par rapport à la première pièce de maintien (22, 44), **en ce que** des éléments de palier de montage (56, 62) sont prévus sur la deuxième pièce de maintien (46) et sur le contenant stérile au voisinage de l'ouverture d'échange de gaz pour le montage mobile de la deuxième pièce de maintien (46) sur le contenant stérile, et **en ce que** le filtre stérile (14), dans la position de base, est maintenu sous précontrainte entre ladite au moins une première et ladite au moins une deuxième surface de maintien (32, 82).

2. Contenant stérile selon la revendication 1, **caractérisé en ce que** ladite au moins une première et ladite au moins une deuxième surface de maintien (32, 82) sont agencées en regard l'une de l'autre.

3. Contenant stérile selon l'une des revendications précédentes, **caractérisé en ce que** le filtre stérile (14) et/ou l'ouverture d'échange de gaz (16) sont d'une configuration de forme circulaire ou sensiblement de forme circulaire.

4. Contenant stérile selon l'une des revendications précédentes, **caractérisé en ce que** l'ouverture d'échange de gaz (16) est prévue sur le couvercle (10), et **en ce que** la surface de paroi (34) forme une partie du couvercle (10) du contenant stérile.

5. Contenant stérile selon l'une des revendications précédentes, **caractérisé en ce que** la première pièce de maintien (22) comporte un logement d'accueil de filtre stérile (42), qui présente une surface de section transversale correspondant sensiblement à une surface de filtre du filtre stérile (14).

6. Contenant stérile selon la revendication 5, **caractérisé en ce que** le logement d'accueil de filtre stérile (42) est réalisé à la manière d'une coupelle.

7. Contenant stérile selon l'une des revendications 5 ou 6, **caractérisé en ce que** dans une paroi latérale (38) du logement d'accueil de filtre stérile (42) sont prévues des ouvertures de passage (112).

8. Contenant stérile selon l'une des revendications précédentes, **caractérisé en ce qu'**il est prévu au moins un élément d'appui (24, 30; 72, 76, 78, 80), qui comprend ladite au moins une première surface de maintien (32) ou ladite au moins une deuxième surface de maintien (82), et qui présente des surfaces d'appui (32, 82) couvrant au moins partiellement l'ouverture d'échange de gaz (16) pour l'appui, d'un côté, du filtre stérile (14).

9. Contenant stérile selon la revendication 8, **caractérisé en ce que** ledit au moins un élément d'appui (24, 30; 72, 76, 78, 80) est une nervure d'appui (24; 72) radiale, issue d'un centre de l'ouverture d'échange de gaz (16).

10. Contenant stérile selon la revendication 8 ou la revendication 9, **caractérisé en ce que** ledit au moins un premier élément d'appui (24, 30; 72, 76, 78, 80) est un anneau d'appui (30; 76, 78, 80) concentrique à l'ouverture d'échange de gaz (16).

11. Contenant stérile selon la revendication 10, **caractérisé en ce que** sont prévus deux ou trois anneaux d'appui concentriques (76, 78, 80).

12. Contenant stérile selon l'une des revendications précédentes, **caractérisé en ce que** ladite au moins une première et/ou ladite au moins une deuxième pièce de maintien (22, 44, 46) portent un joint d'étanchéité (102, 106) contre lequel s'appuie le filtre stérile (14), dans la position stérile.

13. Contenant stérile selon la revendication 12, **caractérisé en ce que** ladite au moins une première et/ou ladite au moins une deuxième pièce de maintien (22, 44, 46) présentent un logement d'accueil de joint d'étanchéité (100, 104) dans lequel s'engage au moins partiellement le joint d'étanchéité (102, 106).

14. Contenant stérile selon l'une des revendications 12 ou 13, **caractérisé en ce que** le joint d'étanchéité (102, 106) est réalisé sous la forme d'un anneau d'étanchéité (102, 106).

15. Contenant stérile selon l'une des revendications précédentes, **caractérisé en ce que** la première pièce de maintien (22, 44) et la deuxième pièce de maintien (46) peuvent être reliées mutuellement de manière amovible.

16. Contenant stérile selon l'une des revendications précédentes, **caractérisé en ce que** le filtre stérile (14) et le système de maintien de filtre (12) peuvent être reliés de manière amovible, et **en ce que** le filtre stérile (14) peut, dans une position de retrait, être détaché du système de maintien de filtre (12), et, dans une position de liaison, est maintenu sur le système de maintien de filtre (12).

17. Contenant stérile selon la revendication 16, **caractérisé en ce qu'**il est prévu un système de liaison à baïonnette (88, 94, 96) pour relier la première pièce de maintien (22, 44) à la deuxième pièce de maintien (46) et pour transférer le système de maintien de filtre (12) de la position de retrait à la position de liaison.

18. Contenant stérile selon l'une des revendications 16 ou 17, **caractérisé en ce qu'**il est prévu un mécanisme de verrouillage (98) destiné à verrouiller la position de liaison des deux pièces de maintien (22, 44, 46).

19. Contenant stérile selon la revendication 18, **caractérisé en ce que** le mécanisme de verrouillage (98) comprend une liaison par encliquetage.

20. Contenant stérile selon l'une des revendications précédentes, **caractérisé en ce qu'**il est prévu un élément de recouvrement (68; 108), qui recouvre le filtre stérile (14) d'un côté.

21. Contenant stérile selon la revendication 20, **caractérisé en ce que** la deuxième pièce de maintien (46) comprend l'élément de recouvrement (68).

22. Contenant stérile selon la revendication 20 ou la revendication 21, **caractérisé en ce que** la deuxième pièce de maintien (46) et l'élément de recouvrement (68) sont réalisés d'un seul tenant.

23. Contenant stérile selon l'une des revendications 20 à 22, **caractérisé en ce que** l'élément de recouvrement (68) est doté d'ouvertures de passage (86) pour permettre une liaison fluidique entre le compartiment d'accueil et le filtre stérile (14).

24. Contenant stérile selon la revendication 23, **caractérisé en ce que** les ouvertures de passage (86) sont recouvertes, dans une direction transversale à une direction de passage, par des éléments de recouvrement d'ouverture de passage (84).

25. Contenant stérile selon l'une des revendications 20 à 24, **caractérisé en ce que** l'élément de recouvrement (68) est espacé du filtre stérile (14), et **en ce que** des éléments d'appui (72, 76, 78, 80) de la première et/ou de la deuxième pièce de maintien (22, 44, 46) forment des entretoises d'écartement (72, 76, 78, 80) pour l'élément de recouvrement (68).

26. Contenant stérile selon l'une des revendications précédentes, **caractérisé en ce que** dans la position de base, le clapet de valve (14) est maintenu sous précontrainte sur le contenant stérile.

27. Contenant stérile selon l'une des revendications précédentes, **caractérisé en ce que** la valve de sureté de pression (14, 16, 44, 46) est réalisée sous la forme d'une valve d'entrée, qui prend la position de passage lorsqu'une pression régnant dans un environnement du contenant stérile, dépasse une pression régnant dans le compartiment d'accueil, d'une valeur correspondant à la différence de pression minimale.

28. Contenant stérile selon l'une des revendications précédentes, **caractérisé en ce que** le filtre stérile (14) forme le clapet de valve (14).

29. Contenant stérile selon l'une des revendications précédentes, **caractérisé en ce qu'**il est prévu au moins une butée (58, 64), qui limite un mouvement d'ouverture du clapet de valve (14) à partir de l'ouverture de valve (16) .

30. Contenant stérile selon l'une des revendications précédentes, **caractérisé en ce que** le filtre stérile (14) est un filtre permanent, notamment fabriqué en polytétrafluoroéthylène (PTFE).

31. Contenant stérile selon l'une des revendications précédentes, **caractérisé en ce que** le couvercle (10) est fabriqué en une matière plastique, notamment en polyétheréthercétone (PEEK) ou polyphénylènesulfone (PPSU).
